# EUROPÄISCHE PATENTSCHRIFT

(11) **EP 0 574 658 B1**
(45) Veröffentlichungstag und Bekanntmachung des Hinweises auf die Patenterteilung: **21.05.1997**
(21) Anmeldenummer: 93104168.5
(22) Anmeldetag: 15.03.1993
(51) Int. Cl.: A61C 8/00

(54) **Einsetzwerkzeug für eine Schraubenezahnprothese**
Implantation tool for a screw-in dental prosthesis
Outil pour implanter une prothèse dentaire à vis

(30) Priorität: 13.06.1992 DE 4219411
(43) Veröffentlichungstag der Anmeldung: 22.12.1993
(73) Patentinhaber: ALTATEC Medizintechnische Elemente GmbH & Co. KG., 75449 Wurmberg (DE); IMZ-Fertigungs- und Vertriebsgesellschaft für dentale Technologie mbH, 70794 Filderstadt (DE)
(72) Erfinder: Dürr, Walter, W-7537 Remchingen (DE); Kirsch, Axel, Dr., W-7024 Filderstadt (DE)
(74) Vertreter: Goddar, Heinz J., Dr.

(56) Entgegenhaltungen:
- DE-A- 2 028 517
- US-A- 3 690 005
- US-A- 4 179 809
- US-A- 4 253 833
- US-A- 4 856 994

## Beschreibung

Die Erfindung betrifft ein Einsetzwerkzeug für eine Schraubeneinheit gemäß dem Oberbegriff von Anspruch 1.

Die Schraubeneinheit nach der DE-C-4 115 961 dient dazu, einen mit Verschlußschraube für das Innengewinde versehenen, im Knochenmaterial zu implantierenden Grundkörper z. B. eines Dentalimplantates während der Implantation sicher und präzise zu handhaben. Der dabei vorgesehene Einbringpfosten, der mit festem, aber lösbarem Sitz an der Verschlußschraube vorgesehen ist, läßt sich mittels eines Spezialwerkzeuges handhaben, wobei hierfür in der DE-C-4 115 961 beispielsweise eine Pinzette genannt ist.

Problematisch kann dabei unter Umständen sein, daß bei unachtsamer Handhabung durch den Operateur der Einbringpfosten nach dem Ablösen von der Verschlußschraube z. B. in der Mundhöhle eines Patienten verlorengeht.

In der US-A-4 253 833 wird das Einsetzwerkzeug von einem von oben auf die Schraubeneinheit aufsetzbaren Greifinstrument gebildet.

Der Erfindung liegt die Aufgabe zugrunde, ein anderes Einsetzwerkzeug zu schaffen, welches die Möglichkeit zu einer unverlierbaren Handhabung des Einbringpfostens gibt.

Erfindungsgemäß wird diese Aufgabe durch das in Anspruch 1 definierte Einsetzwerkzeug gelöst.

Dabei kann vorgesehen sein, daß das Griffteil an seinem der Einsetzgabel abgewandten Ende einen Schlagkopf zur Beaufschlagung mit einem Präzisionshammer oder dergleichen aufweist.

Weiterhin sieht die Erfindung vor, daß die Gabelzinken der Einsetzgabel federnd ausgebildet sind.

Auch kann nach der Erfindung vorgesehen sein, daß in wenigstens einer der Gabelzinken ein in den Gabelspalt Vorspringender Klemmzapfen vorgesehen ist, der in Richtung auf die gegenüberliegende Gabelzinke federbelastet ist.

Dabei schlägt die Erfindung weiterhin vor, daß der Klemmzapfen an einem Ende eines im wesentlichen parallel zu der Gabelzinke verlaufenden Schwingarmes angeordnet ist, dessen anderes Ende an der Gabelbasis befestigt ist.

Auch kann hierbei vorgesehen sein, daß der Schwingarm in einem parallel zur Gabelebene verlaufenden Längsschlitz der Gabelzinke liegt.

Eine weitere Ausführungsform der Erfindung sieht vor, daß die den Gabelspalt begrenzenden, zur Gabelebene im wesentlichen senkrechten Flächen der Gabelzinken und/oder der Gabelbasis von der dem Griffteil zugewandten zu der dem Griffteil abgewandten Seite der Einsetzgabel den Gabelspalt verengend leicht angeschrägt verlaufen.

Auch kann nach der Erfindung vorgesehen sein, daß das Griffteil einen ersten Abschnitt, der im wesentlichen senkrecht zur Ebene der Einsetzgabel verläuft, einen zweiten Abschnitt, der gegenüber dem ersten Abschnitt spitzwinklig abgeknickt ist, und einen dritten Abschnitt aufweist, der im wesentlichen parallel zum ersten Abschnitt verläuft.

Dabei sieht die Erfindung gegebenenfalls auch vor, daß mindestens zwei der Abschnitte des Griffteiles lösbar miteinander verbunden sind.

Die Erfindung kann ferner dadurch gekennzeichnet sein, daß die Einzelelemente des Griffteiles und/oder der Einsetzgabel aus Metall bestehen.

Dadurch, daß bei der Erfindung ein federndes Halten des Einbringpfostenschaftes mittels der Einsetzgabel gewährleistet ist, kann der Einbringpfosten nach dem Abnehmen von der Verschlußschraube in z. B. der Mundhöhle des Patienten nicht verlorengehen. Auch werden die Schlagkräfte, welche über das Einsetzwerkzeug auf die Verschlußschraube und damit auf den zu implantierenden Grundkörper übertragen werden, nicht auf den Kopf des Einbringpfostens ausgeübt, wie dies bei der Schraubeneinheit nach der DE-C-4 115 961 der Fall ist, sondern über die Gabelzinken der Einsetzgabel auf die Verdickung des Einbringpfostens, von wo sie auf die Verschlußschraube und damit auf den Grundkörper übertragen werden, so daß das Aufbringen von Schermomenten auf den Einbringpfosten, welches zu einem unbeabsichtigten Abknicken des Einbringpfostens von der Verschlußschraube vor vollständigen Implantieren des Grundkörpers fuhren könnte, verhindert wird.

Weitere Merkmale und Vorteile der Erfindung ergeben sich aus der nachstehenden Beschreibung, in der ein Ausführungsbeispiel anhand der schematischen Zeichnung im einzelnen erläutert ist. Dabei zeigt:
- Fig. 1: ein Ausführungsbeispiel eines Einsetzwerkzeuges nach der Erfindung maßstäblich in der Seitenansicht;
- Fig. 2: das Einsetzwerkzeug von Figur 1 in Figur 1 entsprechender, jedoch vergrößerter Darstellung;
- Fig. 3: das Einsetzwerkzeug von Figur 2 in der Vorderansicht, in Figur 2 von links gesehen; und
- Fig. 4: das Ausführungsbeispiel von Figur 2 und Figur 3 im Schnitt entlang der Linie IV - IV von Figur 2, in Richtung der Pfeile von unten gesehen.

Wie die Zeichnung erkennen läßt, weist das Einsetzwerkzeug nach der Erfindung ein langgestrecktes Griffteil 10 auf, welches im Anschluß an eine Einsetzgabel 12 einen im wesentlichen senkrecht zu deren Ebene verlaufenden ersten Abschnitt 14, einen spitzwinklig hiergegen abgeknickten zweiten Abschnitt 16 und einen hieran anschließenden, im wesentlichen parallel zum ersten Abschnitt 14 verlaufenden dritten Abschnitt 18 hat. An dem der Einsetzgabel 12 abgewandten Ende des dritten Abschnittes befindet sich ein Schlagkopf 20. Der zweite Abschnitt 16 ist mit dem dritten Abschnitt 18 lösbar verbunden, z. B. verschraubt. Auch zwischen der Gabel 12 und dem ersten Abschnitt 16 kann eine lösbare Verbindung vorgesehen sein. Die Außenfläche des dritten Abschnittes 18, welcher bei dem gezeigten Ausführungsbeispiel einen größeren Außendurchmesser hat als der zweite Abschnitt 16, der wiederum dicker ausgebildet ist als der erste Abschnitt 14, ist bei dem gezeigten Ausführungsbeispiel mit polygonalen Halteflächen versehen, um auf diese Weise das Erfassen durch den Operateur zu erleichtern.

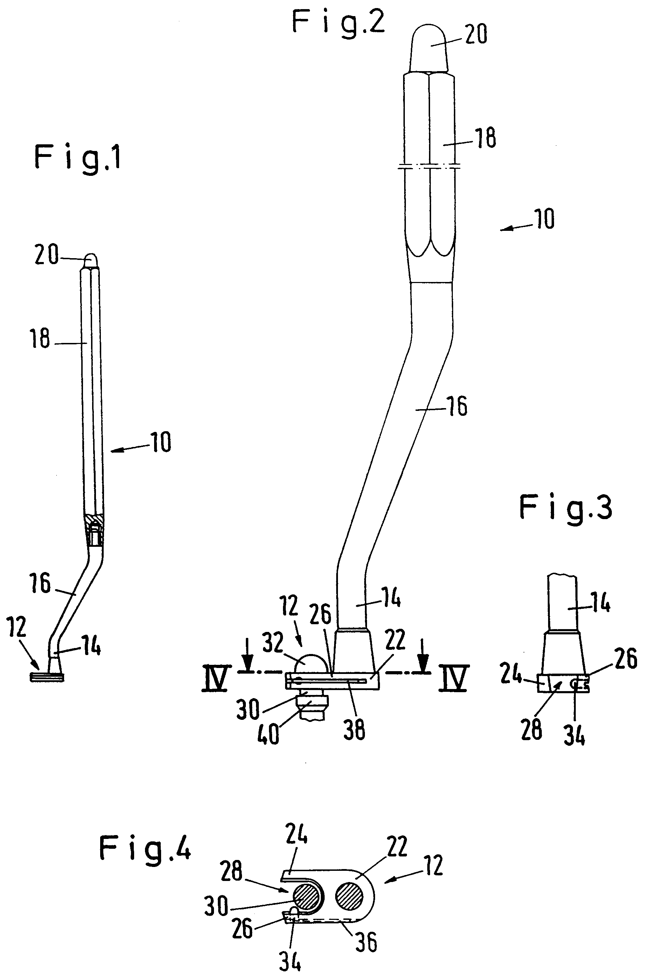

Die Einsetzgabel 12 weist eine Gabelbasis 22 und zwei Gabelzinken 24, 26 auf, die einen Gabelspalt 28 begrenzen. Die Weite des Gabelspaltes 28, d. h. der gegenseitige Abstand der Gabelzinken 24, 26, ist geringfügig größer als der Außendurchmesser eines Einbringpfostenschaftes 30, jedoch kleiner als der Außendurchmesser eines Einbringpfostenkopfes 32 des Einbringpfostens einer Schraubeneinheit, wie sie in der DE-PS 41 15 961 (nicht vorveröffentlicht) beschrieben ist. In den Gabelspalt 28 springt ein Klemmzapfen 34 vor, der, senkrecht hierzu angeordnet, am freien Ende eines Schwingarmes 36 sitzt, dessen gegenüberliegendes Ende, wie Figur 4 erkennen läßt, mit der Gabelbasis fest verbunden ist. Der Schwingarm 36 sitzt in einem zur Gabelebene parallelen Längsschlitz 38 der Gabelzinke 26 und ist somit in Richtung auf die gegenüberliegende Gabelzinke 24 mittels des Schwingarmes 36 federbelastet. Der Abstand zwischen dem dem Schwingarm 36 abgewandten Ende des Klemmzapfens 34 und der gegenüberliegenden Fläche der Gabelzinke 24 ist dabei kleiner als der Außendurchmesser des Einbringpfostenschaftes 30. Die den Gabelspalt 28 begrenzenden Flächen der Gabelzinken 24, 26 bzw. der Gabelbasis 22 sind in Richtung von der dem Griffteil 10 zugewandten Seite zu der dem Griffteil 10 abgewandten Seite leicht angeschrägt. Sämtliche Einzelteile des vorstehend beschriebenen Einsetzwerkzeuges sind bei dem gezeigten Ausführungsbeispiel aus Metall hergestellt.

Das Einsetzwerkzeug wird wie folgt verwendet:

Die Einsetzgabel 12 wird von dem Operateur über den Einbringpfostenschaft 30 der Schraubeneinheit geschoben, mittels welcher das Innengewinde des nicht gezeigten Grundkörpers eines Implantates, z. B. eines Dentalimplantates, verschlossen ist, wobei wegen der diesbezüglichen Einzelheiten auf die DE-PS 41 15 961 verwiesen wird. Der Klemmzapfen 34 greift dabei federnd an der der Gabelbasis 22 abgewandten Seite hinter den Einbringpfostenschaft 30, so daß daraufhin der Einbringpfostenschaft 30 durch die Einsetzgabel 12 unverlierbar gehalten ist, da der Einbringpfostenkopf 32 und eine an dem dem Einbringpfostenkopf 32 abgewandten Ende des Einbringpfostenschaftes 30 vorgesehene Verdickung 40 einen Außendurchmesser haben, der größer ist als die Weite des Gabelspaltes 28.

Anschließend wird der auf diese Weise mit dem Einsetzwerkzeug lösbar, jedoch unverlierbar verbundene Grundkörper des Implantates vom Operateur in eine vorbereitete Bohrung des Kieferknochens oder dergleichen eingesetzt und dadurch in diese eingeführt, daß auf den Schlagkopf 20 mit einem Hammer oder einem anderen geeigneten Fein-Schlagwerkzeug eingewirkt wird. Die Einsetzgabel 12 überträgt die entsprechenden Schlagkräfte auf die Verdickung 40 und damit in der aus der DE-PS 41 15 961 ersichtlichen Weise auf den Grundkörper. Nach Beendigung des Einbringens des Grundkörpers in den Kieferknochen oder dergleichen wird der Einbringpfosten der Verschlußschraube, wie in der DE-PS 41 15 961 beschrieben, dessen Bestandteile der Einbringpfostenkopf 32, der Einbringpfostenschaft 30 und die Verdickung 40 sowie ein zeichnerisch in Figur 2 nicht dargestelltes Übergangsteil bilden, in der in der DE-PS 41 15 961 beschriebenen Weise von der Verschlußschraube, die im Grundkörper vorläufig verbleibt, gelöst, wobei der Einbringpfosten unverlierbar mittels des Einsetzwerkzeuges nach der Erfindung gehalten wird und z. B. aus der Mundhöhle des Patienten entfernt werden kann.

## Patentansprüche

1. Einsetzwerkzeug für eine Schraubeneinheit mit einer einen Schraubschlitz aufweisenden Verschlußschraube zum aseptischen Verschließen eines Innengewindes eines in einem Knochenmaterial zu implantierenden Grundkörpers, wobei mit der Verschlußschraube lösbar ein Einbringpfosten verbunden ist, der einen im wesentlichen zylindrischen Einbringpfostenschaft aufweist, an dessen der Verschlußschraube abgewandtem Ende ein Einbringpfostenkopf mit gegenüber dem Einbringpfostenschaft vergrößertem Durchmesser und an dessen der Verschlußschraube zugewandtem Ende eine Einbringpfostenverdickung mit gegenüber dem Einbringpfostenschaft vergrößerten Durchmesser vorgesehen sind, mit einem langgestreckten Griffteil (10), dadurch gekennzeichnet, daß das Einsetzwerkzeug an seinem Arbeitsende eine im wesentlichen senkrecht zu seiner Längsachse vorspringende zweizinkige Einsetzgabel (12) zum Untergreifen des Einbringpfostenkopfes (32) mit einem Gabelspalt (28) aufweist, dessen Weite im wesentlichen gleich dem, höchstens aber geringfügig größer als der Außendurchmesser des Einbringpfostenschaftes (30) ist, und daß die Einsetzgabel Mittel zum vorübergehenden Einklemmen des Einbringpfostenschaftes (30) aufweist.

2. Einsetzwerkzeug nach Anspruch 1, dadurch gekennzeichnet, daß das Griffteil (10) an seinem der Einsetzgabel (12) abgewandten Ende einen Schlagkopf (20) zur Beaufschlagung mit einem Präzisionshammer oder dergleichen aufweist.

3. Einsetzwerkzeug nach Anspruch 1 oder 2, dadurch gekennzeichnet, daß die Gabelzinken (24, 26) der Einsetzgabel (12) federnd ausgebildet sind.

4. Einsetzwerkzeug nach Anspruch 1 oder 2, dadurch gekennzeichnet, daß in wenigstens einer der Gabelzinken (24, 26) ein in den Gabelspalt (28) vorspringender Klemmzapfen (34) vorgesehen ist, der in Richtung auf die gegenüberliegende Gabelzinke federbelastet ist.

5. Einsetzwerkzeug nach Anspruch 4, dadurch gekennzeichnet, daß der Klemmzapfen (34) an einem Ende eines im wesentlichen parallel zu der Gabelzinke (24, 26) verlaufenden Schwingarmes (36) angeordnet ist, dessen anderes Ende an der Gabelbasis (22) befestigt ist.

6. Einsetzwerkzeug nach Anspruch 5, dadurch gekennzeichnet, daß der Schwingarm (36) in einem parallel zur Gabelebene verlaufenden Längsschlitz (38) der Gabelzinke (24, 26) liegt.

7. Einsetzwerkzeug nach einem der vorangehenden Ansprüche, dadurch gekennzeichnet, daß die den Gabelspalt (28) begrenzenden, zur Gabelebene im wesentlichen senkrechten Flächen der Gabelzinken (24, 26) und/oder der Gabelbasis (22) von der dem Griffteil (10) zugewandten zu der dem Griffteil (10) abgewandten Seite der Einsetzgabel (12) den Gabelspalt (28) verengend leicht angeschrägt verlaufen.

8. Einsetzwerkzeug nach einem der vorangehenden Ansprüche, dadurch gekennzeichnet, daß das Griffteil (10) einen ersten Abschnitt (14), der im wesentlichen senkrecht zur Ebene der Einsetzgabel (12) verläuft, einen zweiten Abschnitt (16), der gegenüber dem ersten Abschnitt (14) spitzwinklig abgeknickt ist, und einen dritten Abschnitt (18) aufweist, der im wesentlichen parallel zum ersten Abschnitt (14) verlauft.

9. Einsetzwerkzeug nach Anspruch 8, dadurch gekennzeichnet, daß mindestens zwei der Abschnitte (14, 16, 18) des Griffteiles (10) lösbar miteinander verbunden sind.

10. Einsetzwerkzeug nach einem der vorangehenden Ansprüche, dadurch gekennzeichnet, daß die Einzelelemente des Griffteiles (10) und/oder der Einsetzgabel (12) aus Metall bestehen.

## Claims

1. An implantation tool for a screw unit comprising a closure screw with a slot for aseptically closing or sealing an internal thread on a base member for implanting in a bone material, the screw being releasably connected to an insertion post having a substantially cylindrical shank with a larger-diameter head at the end remote from the screw and a larger-diameter thick part at the end facing the screw and with an elongate handle part (10), characterised in that the implantation tool at its working end has a two-pronged fork (12) projecting substantially at right angles to its longitudinal axis and for gripping underneath the insertion-post head (32), the fork having a gap (28) with a width substantially equal to or at most slightly more than the outside diameter of the insertion-post shank (30), and the implantation fork has means for temporarily clamping the insertion-post shank (30).

2. An implantation tool according to claim 1, characterised in that the handle part (10) at its end remote from the implantation fork (12) has a head (20) for striking with a precision hammer or the like.

3. An implantation tool according to claim 1 or 2, characterised in that the prongs (24, 26) of the fork (12) are resilient.

4. An implantation tool according to claim 1 or 2, characterised in that a clamping pin (34) is disposed in at least one of the prongs (24, 26) and projects into the gap (28) and is spring-loaded in the direction towards the opposite prong.

5. An implantation tool according to claim 4, characterised in that the clamping pin (34) is disposed on one end of a swing arm (36) extending substantially parallel to the prong (24, 26) and having its other end secured to the base (22) of the fork.

6. An implantation tool according to claim 5, characterised in that the swing arm (36) rests in a slot (38) in the prong (24, 26) parallel to the plane of the fork.

7. An implantation tool according to any of the preceding claims, characterised in that the base (22) and/or the surfaces of the prongs (24, 26) defining the gap (28) and substantially perpendicular to the fork plane are slightly chamfered and narrow the gap (28) from the side of the fork (12) facing the handle part (10) to the side remote from the handle part (10).

8. An implantation tool according to any of the preceding claims, characterised in that the handle part (10) has a first portion (14) substantially at right angles to the plane of the fork (12), a second portion (16) bent at an acute angle to the first portion (14), and a third portion (18) substantially parallel to the first portion (14).

9. An implantation tool according to claim 8, characterised in that at least two of the portions (14, 16, 18) of the handle part (10) are releasably connected.

10. An implantation tool according to any of the preceding claims, characterised in that the individual components of the handle part (10) and/or of the implantation fork (12) are made of metal.

## Revendications

1. Outil d'implantation pour une unité à vis, comportant une vis d'obturation présentant une fente et destinée à l'obturation aseptique d'un filet intérieur d'un corps de base à implanter dans une matière osseuse, une colonnette de pose étant reliée de manière détachable à la vis d'obturation, cette colonnette comprenant une tige de colonnette de pose, sensiblement cylindrique, dont l'extrémité, dirigée à l'opposé de la vis d'obturation, présente une tête de colonnette de pose ayant un diamètre plus grand que celui de la tige de colonnette de pose, l'autre extrémité de celle-ci, tournée vers la vis d'obturation, présentant un épaississement de colonnette de pose ayant un diamètre augmenté par rapport à celui de la tige de colonnette de pose, l'outil comportant une partie préhension (10) très allongée et étant caractérisé en ce que l'outil d'implantation présente, à son extrémité de travail, une fourche (12) à deux fourchons, qui est en saillie sensiblement perpendiculairement à l'axe longitudinal de l'outil et qui est destinée à saisir par dessous la tête de colonnette de pose (32) par un entredent (28) dont la largeur est sensiblement égale, mais au maximum légèrement supérieure, au diamètre extérieur de la tige de colonnette de pose (30), et en ce que la fourche d'implantation présente des moyens pour serrer provisoirement la tige de colonnette de pose (30).

2. Outil d'implantation selon la revendication 1, caractérisé en ce que la partie préhension (10) présente, à son extrémité dirigée à l'opposé de la fourche d'implantation (12), une tête de percussion (20) à frapper au moyen d'un marteau de précision ou analogue.

3. Outil d'implantation selon la revendication 1 ou 2, caractérisé en ce que les fourchons (24, 26) de la fourche d'implantation (12) sont de conception élastique.

4. Outil d'implantation selon la revendication 1 ou 2, caractérisé en ce qu'il est prévu, dans au moins l'un des fourchons (24, 26), un téton de serrage (34) en saillie dans l'entredent (28) et élastiquement sollicité en direction du fourchon situé en face.

5. Outil d'implantation selon la revendication 4, caractérisé en ce que le téton de serrage (34) est situé au niveau d'une extrémité d'un bras oscillant (36), qui s'étend sensiblement parallèlement au fourchon (24, 26) et dont l'autre extrémité est fixée à la base (22) de la fourche.

6. Outil d'implantation selon la revendication 5, caractérisé en ce que le bras oscillant (36) est placé dans une fente longitudinale (38) du fourchon (24, 26), qui s'étend parallèlement au plan de la fourche.

7. Outil d'implantation selon l'une des revendications précédentes, caractérisé en ce que les faces des fourchons (24, 26) et/ou de la base (22) de la fourche, définissant l'entredent (28) et sensiblement perpendiculaires au plan de la fourche, s'étendent légèrement en biais depuis le côté de la fourche d'implantation (12) tourné vers la partie préhension (10) jusqu'au côté de la fourche dirigé à l'opposé de la partie préhension (10), pour ainsi rétrécir progressivement l'entredent (28).

8. Outil d'implantation selon l'une des revendications précédentes, caractérisé en ce que la partie préhension (10) présente un premier tronçon (14), qui s'étend sensiblement perpendiculairement au plan de la fourche d'implantation (12), un deuxième tronçon (16), qui s'étend obliquement par rapport au premier tronçon (14) en formant un angle aigu, et un troisième tronçon (18), qui s'étend sensiblement parallèlement au premier tronçon (14).

9. Outil d'implantation selon la revendication 8, caractérisé en ce qu'au moins deux des tronçons (14, 16, 18) de la partie préhension (10) sont reliés l'un à l'autre de façon détachable.

10. Outil d'implantation selon l'une des revendications précédentes, caractérisé en ce que les éléments individuels de la partie préhension (10) et/ou de la fourche d'implantation (12) sont à base de métal.
